# EUROPEAN PATENT SPECIFICATION

(11) **EP 0 471 114 B1**
(45) Date of publication and mention of the grant of the patent: **09.04.1997**
(21) Application number: 90125479.7
(22) Date of filing: 03.01.1991
(51) Int. Cl.: A61F 13/15

(54) **Thin sanitary napkin**
Dünne Damenbinde
Serviette hygiénique mince

(30) Priority: 19.07.1990 US 556694
(43) Date of publication of application: 19.02.1992
(73) Proprietor: KIMBERLY-CLARK CORPORATION, Neenah, Wisconsin 54956-0349 (US)
(72) Inventor: Serbiak, Paul John, Appleton, WI 54915 (US); King, David Russell, Neenah, WI 54956 (US); Mitchler, Patricia Ann, Neenah, WI 54956 (US); Romans-Hess, Alice Yvonne, Fremont, WI 54940 (US); Van Den Bogart, Thomas William, Kimberly, WI 54136 (US); Peerenboom, Robert John, Little Chute, WI 54140 (US); Finch, Valerie Victoria, Neenah, WI 54956 (US); Hlaban, James John, Neenah, WI 54956 (US)
(74) Representative: Diehl, Hermann O. Th., Dr.

(56) References cited:
- EP-A- 0 122 042
- EP-A- 0 146 190
- EP-A- 0 165 807
- EP-A- 0 202 127
- EP-A- 0 248 584
- EP-A- 0 336 578
- US-A- 4 079 739
- US-A- 4 381 783
- US-A- 4 559 050

## Description

This invention relates to a thin sanitary napkin and a method of manufacturing the napkin.

Sanitary napkins, also referred to as catamenial or feminine pads, are designed to be worn by a female to absorb medium to heavy flow of body fluids such as menses, blood, urine, and other excrements discharged by the body during a menstrual period. Sanitary napkins are external devices which are designed to be aligned approximate the pudendum region of the human body and are generally held in position by being adhesively or mechanically attached to an undergarment. Such products differ from tampons which are classified as internal devices and are designed to be physically inserted into the vaginal cavity. Sanitary napkins also differ from pantiliners and panty shields in several noticeable ways. Sanitary napkins are generally larger in size, have a more defined three-dimensional configuration, are thicker in caliper and are bulkier in appearance than pantiliners or panty shields. Functionally, sanitary napkins are different in that they are constructed to absorb a greater quantity of body fluid and are designed so that they can be worn for a longer period of time; for example, overnight if needed.

Since sanitary napkins are normally used during the major discharge portion of a menstrual period, they are constructed to handle medium to heavy flows and commonly have a total absorbent capacity in the range of about 20 to 50 grams of fluid. Pantiliners and panty shields, on the other hand, are designed to absorb relatively small amounts of body fluids and are marketed to be used at the beginning and end of a menstrual period when flow is light or spotty. Commercially available pantiliners and panty shields are constructed to have a total absorbent capacity in the range of about 1 to 15 grams of fluid.

Today's sociological changes have enabled women to become more active in sports and other types of physical activity. These changes have been complemented by a change in attire and have given women the option of wearing tight, body-fitting clothing. Current sanitary napkins, having a caliper of 6.4 mm or greater, can present an unsightly bulge adjacent the pudendum when worn inside tight-fitting shorts or pants. The overall size and configuration of the napkin can also restrict leg movement or cause discomfort when a women participates in physical or sporting events. In view of this, there is a real need to develop a thin sanitary napkin which is less than about 5 millimeters in caliper yet able to absorb as much body fluid as current available products.

In developing a thin sanitary napkin less than about 5 millimeters in caliper, it was realized that such products had a tendency to twist and bunch when worn. The squeezing of the napkin between the thighs and the resulting deformation as a woman moves about, causes the upper surface of the napkin to acquire a curved or convex shape. This twisting and bunching is referred to as "roping" because a cylindrical profile can be imparted to the sanitary napkin. This roping effect is detrimental because the napkin is unable to absorb body fluid that contacts its upper surface. The fluid discharged from the vagina has a tendency to run off the roped napkin before it can be absorbed and, therefore, the fluid leaks onto the undergarment. This run-off becomes significant during periods of heavy flow.

It has been found that by positioning an absorbent strip, which is stiffer and more absorbent than adjacent portions, along the longitudinal central axis of the sanitary napkin, this roping phenomenon can be mitigated and/or eliminated.

Other manufacturers have recognized the need for a thin sanitary napkin but have not addressed the roping problem. U.S. 4,950,264 and EP-A-0 336 578 issued to Thomas W. Osborn, III, teach a thin sanitary napkin. Osborn teaches a thin sanitary napkin comprised of an absorbent and a liquid-impermeable barrier. This reference teaches that the sanitary napkin has a flexure-resistance of less than about 400 grams, a test capacity of at least about 8.0 grams, and a total capacity of at least about 14.0 grams. Osborn also teaches that the thin sanitary napkin is under 5.0 millimeters in caliper and has a capacity sufficient to absorb medium or heavy flows. However, Osborn does not teach the presence of a longitudinally-extending central absorbent zone, which is less than about 5.08 cm (2 inches) wide, and which is stiffer and more absorbent than adjacent portions of the napkin.

U.S. 4,079,739 issued to Howard A. Whitehead and assigned to the present assignee teaches a feminine pad having a thick central layer of absorbent material. The thick central layer does not contain superabsorbent as does the laminate taught in the present invention.

Document EP-A-0 248 584 (Kao) discloses a sanitary napkin comprising a liquid-permeable surface material, a liquid-impermeable leakproof material and an absorber inserted between said surface material and said leakproof material. The absorber according to document EP-A-0 248 584 comprises a first absorbent paper, a second absorbent paper and an absorbent paper material inserted between both papers whereby said first absorbent paper covers at least a part of said second absorbent paper and is fixed at a portion thereof covering the second absorbent paper onto said leakproof material. According to document EP-A-0 248 584, this structure leads to a sanitary napkin which is protected from deformation and twisting in service. Especially, the shift and twist of the absorber is remarkably reduced.

Other patents, which teach various aspects of this invention, include the following: U.S. 3,065,751 issued to Gobbo et al. which teaches a disposable diaper having a certain Gurley stiffness and having a flexible absorbent layer; Reissue 32,649 issued to Brandt et al. which teaches hydrogel forming polymer compositions for use in absorbent structures and at column 18, lines 40-43, mentions the thickness of the absorbent core; and U.S. 4,578,068 issued to Kramer et al. which teaches an absorbent laminate structure containing superabsorbent particles.

The present invention intends to provide a thin but effective and safe sanitary napkin and a method of making same. This object is solved by the sanitary napkin of independent claims 1 and 3. Further advantageous features of the sanitary napkin are evident from the dependent claims, the description and the drawings. The claims are intended to be understood as a first non-limiting attempt at defining the invention in general terms.

Now a thin sanitary napkin has been developed which has a longitudinally-extending central absorbent zone which is less than 75% of the width of the entire absorbent and less than 60% of the width of said napkin and which is stiffer and more absorbent than adjacent portions of the napkin.

This invention provides a thin sanitary napkin which has a caliper of less than about 5 millimeters and which has a longitudinally-extending central absorbent zone which is more absorbent and stiffer than adjacent zones so as to resist twisting and bunching during use.

Briefly, this invention relates to a thin sanitary napkin designed to be worn by a female to absorb body fluids such as menses, blood, urine, and other excrements discharged during a menstrual period. The sanitary napkin has a liquid-impermeable baffle, an absorbent retained on the baffle, and a caliper of less than about 5 millimeters. The absorbent has a longitudinally-extending central absorbent zone with a width of less than about 2 inches (50.8 mm). The central zone is capable of absorbing at least 20 grams of body fluid which is discharged from the vaginal cavity and deposited onto the napkin. The central zone also has a greater stiffness than adjacent portions of the napkin so as to resist twisting and bunching during use. A method of manufacturing the sanitary napkin is also disclosed.

The general aspect of this invention is to provide a thin sanitary napkin which has a longitudinally-extending central absorbent zone with a width of less than about 2 inches. A more specific object of this invention is to provide a thin sanitary napkin which has a caliper of less than about 5 millimeters and which has a longitudinally-extending central absorbent zone which is more absorbent than adjacent zones and stiffer, so as to resist twisting and bunching during use.

Another aspect of this invention is to provide a method of manufacturing a thin sanitary napkin which is less noticeable when worn under tight-fitting clothing.

A further aspect of this invention is to provide a sanitary napkin which uses less material and therefore is more environmentally friendly.

Still another aspect of this invention is to provide a sanitary napkin which is lower in cost by selectively placing expensive material, such as the superabsorbent and the transfer material, within the napkin where they are most effective while minimizing the amount of material needed.

Still another aspect of this invention is to provide a thin sanitary napkin which has at least three lateral zones which vary in caliper, stiffness and absorbency.

Still further, an aspect of this invention is to provide a thin sanitary napkin which has a relatively stiff central absorbent zone which resist twisting and bunching when worn.

Other aspects and advantages of the present invention will become more apparent to those skilled in the art in view of the following description and the accompanying drawings.

Fig. 1 is a perspective view of a sanitary napkin having a longitudinally-extending central absorbent zone.

Fig. 2 is a cross-sectional view of the sanitary napkin shown in Fig. 1 taken along line 2--2.

Fig. 3 is a cross-sectional view of a sanitary napkin showing an alternative embodiment wherein the absorbent is a laminate-containing superabsorbent.

Fig. 4 is a cross-sectional view of a sanitary napkin showing another embodiment which is similar to Fig. 3 except that a cover is positioned over the absorbent.

Fig. 5 is a cross-sectional view of a sanitary napkin depicting an absorbent comprised of a superabsorbent layer and a pulp layer sandwiched between a cover and a baffle.

Fig. 6 is a cross-sectional view of a sanitary napkin depicting an absorbent comprised of a superabsorbent wrapped in a meltblown web and sandwiched between a cover and a baffle.

Fig. 7 is a perspective view of a sanitary napkin having a longitudinally-extending central absorbent zone enclosed between two tissue layers and aligned below a transfer layer.

Fig. 8 is a cross-sectional of the sanitary napkin shown in Fig. 7 taken along line 8--8.

Fig. 9 is a perspective view of a sanitary napkin having a racetrack profile and having an absorbent with an hourglass profile. The absorbent extends longitudinally along essentially the entire length of the napkin and is located between a transfer layer and a wicking layer.

Fig. 10 is a cross-sectional view of the sanitary napkin shown in Fig. 9 taken along line 10--10.

Fig. 11 is a top view of a sanitary napkin having a pair of wings attached to the longitudinal sides thereof.

Fig. 12 is a flow diagram of a method used to manufacture the sanitary napkin of this invention.

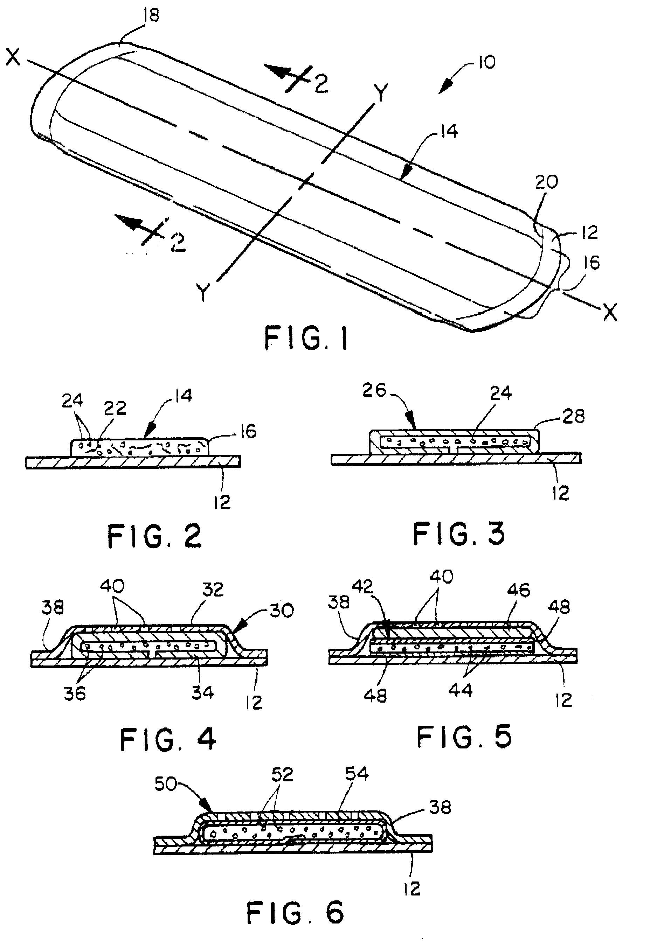

Referring to Fig. 1, a thin sanitary napkin 10 is shown which is designed to be worn by a female to absorb body fluids such as menses, blood, urine, and other excrements discharged during a menstrual period. The sanitary napkin 10 is about 150 mm to 320 mm long, about 60 mm to 120 mm wide and has a racetrack shape with rounded ends. The napkin 10 has a caliper or thickness of less than about 5 millimeters, preferably less than about 4 millimeters, and most preferably less than about 3 millimeters. The sanitary napkin 10 includes a liquid-impermeable baffle 12 and an absorbent 14 which is secured to or retained on the baffle 12.

The liquid-impermeable baffle 12 is designed to face the inner surface, generally the crotch portion, of an undergarment (not shown). The baffle 12 permits the passage of air or vapor out of the sanitary napkin 10 while blocking the passage of body fluids and liquids. The baffle 12 can be made from a micro-embossed polymeric films such as polyethylene or polypropylene, or it can be made from bicomponent films. A preferred material is polyethylene film.

The absorbent 14 can be secured to the baffle 12 by an adhesive or, alternatively, it can be enclosed by a liquid-permeable cover. The absorbent 14 has a longitudinally-extending central absorbent zone 16 aligned along the longitudinal axis, designated X--X, of the napkin 10. The central absorbent zone 16 has an overall length which extends at least about 50%, and preferably about 75%, of the length of the napkin 10. More preferably, the central absorbent zone 16 extends essentially the entire length of the napkin 10 and terminates at the end seal lines 18 and 20. The central absorbent zone 16 has a width of less than about 2 inches (50.8 mm), preferably between about 0.5 and 2.0 inches (12.7 mm and 50.8 mm), and most preferably about 1.25 inches (31.8 mm). The central absorbent zone 16 can have a width which is equal to or less than the width of the absorbent 14 when measured across the central transverse axis Y--Y of the napkin 10. Preferably, the central absorbent zone 16 has a width which is less than about 60% of the width of the sanitary napkin 10 when measured across the narrowest portion of the napkin 10. The central absorbent zone 16 also has a width which is preferably less than about 75% of the width of the absorbent 14 when measured across the narrowest portion of the absorbent 14.

The central absorbent zone 16 represents the significant absorbing portion of the napkin 10 and has the capability of absorbing at least about 80%, preferably about 90%, and most preferably about 95% of the body fluid deposited onto the napkin 10. In terms of amount of body fluid, the central zone 16 can absorb at least 20 grams, preferably about 25 grams, and most preferably, about 30 or more grams of body fluid. It should be noted that the amount of body fluid which can be absorbed by the sanitary napkin 10 can be determined using a saline solution.

The capacity of the sanitary napkin 10 can be determined by the following test. In performing this test, one napkin is sufficient. The sanitary napkin to be tested is first conditioned by leaving it in a room which is at 21 ± 1° C and at 50 ± 2% relative humidity for a period of two hours. If the napkin contains a peel strip, this is removed. The entire napkin, minus any peel strip, is weighed to the nearest 0.1 gram. The napkin is then submerged in a beaker of stabilized isotonic saline which contains no preservatives. A suitable sterile saline is commercially sold by Baxter Travenol Company of Deerfield, Illinois under catalog no. B3158-2. The napkin is totally submerged and is not bent or otherwise twisted or folded. The napkin is submerged for 10 minutes. The napkin is removed from the saline and suspended for two minutes in a vertical position to allow the saline to drain out of the napkin. The napkin is then placed with the bodyside cover face down on an absorbent blotter. The blotter can be filter paper no. ED 631-25 available from the Ahlstrom Filtration Inc., Mount Holly Springs, Pennsylvania 17065. A uniform 17.6 grams per square centimeter load is placed over the napkin to squeeze out excess fluid. The absorbent blotter is replaced every 30 seconds until the amount of fluid transferred to the absorbent blotter is less than 0.5 grams in a 30 second period. Next, the napkin is weighed to the nearest 0.1 gram and the dry weight of the napkin is subtracted. The difference in grams is the capacity of the napkin.

The central absorbent zone 16 is also stiffer than adjacent, longitudinally-extending zones. This stiffness can be obtained by making the central zone 16 thicker; by constructing it out of several layers, by using stiffer materials, by changing the basis weight or by placing another layer of material vertically adjacent to it. Preferably, the central absorbent zone has a Gurley stiffness of at least about 500 milligrams, and preferably higher. See Table 1 below.

The ability of certain zones of a sanitary napkin to resist an applied bending force, known as pad stiffness, is determined by measuring the amount of force required to bend a rectangular composite sample cut from the sanitary napkin that includes all layers excluding the peel strip. The force needed to bend each sample is measured using a Gurley Model 4171-d Digital Stiffness Tester which along with weights and precalibration strips are available through Teledyne Gurley, Troy, New York. The Gurley stiffness test procedure is modeled after the Technical Association of the Pulp and Paper Industry (TAPPI) method T 543 pm-84. The Gurley Digital Stiffness Tester is an instrument consisting of a balanced vane, which is center-pivoted, and to which a variety of weights can be added below its pivot point. The vane moves freely to accommodate testing in both left and right directions which would be analogous to upward and outward body flexing of the samples.

There is a two part calibration to the Gurley Stiffness Tester. The first calibration is done to ensure that the "Vane" pendulum is swinging according to specification against a known material (i.e., a brass strip). The Gurley instrument is calibrated following the Gurley Digital Stiffness Tester Instruction Manual to within 5% variation with a 50.8 mm wide by 25.4 mm long precalibrated Brass Calibration Strip, Gurley part no. 31644. The second calibration is done to ensure that the internal electronic calculations and conversions are accurate.

The samples cut from each sanitary napkin are 12.7 mm ± 0.4 mm wide by 25.4 mm ± 0.4 mm long. Each sample overlaps the top of the Gurley vane by 6.4 mm. During a test, the sample is moved against the top edge of the vane until the sample bends and the vane releases contact with the bottom edge of the sample. The point of release is measured by an electronic optical encoder which provides a greater degree of accuracy over the earlier model Gurley Stiffness Tester as was used in TAPPI T 543 pm-84. The electronic optical encoder also displays the result on the digital readout. The readout continuously displays readings from tests performed in both the left and right directions. The Gurley Model 4171-d also computes automatically through an internal microprocessor and displays the average of left and right bending stiffness data after each measurement. The average reading is then converted by this Gurley instrument into milligrams of Gurley stiffness relative to a sample size of 24.5 mm wide by 76.2 mm long.

The Gurley Stiffness Tester should be set up as follows. The required weight is attached and the base of the instrument is levelled by adjusting the leveling screw until the level's bubble is centered and the pendulum's pointer is indicating zero. The switches are set to correspond to the weight being used, the weight's position on the pendulum, the width of the specimen being tested, and the length of the specimen. For example: if a 25.4 mm x 12.7 mm specimen is tested with the 5 gm weight in the 25.4 mm slot, the switches would be set as follows:

| | |
|---|---|
| Weight | = 5 gm |
| Weight Position | = 1 inch |
| Width | = .5 inch |
| Length | = 1 inch |

The test procedure to be performed is as follows:
1. Center the specimen strip over the pendulum such that exactly 6.4 mm (0.25 inches) overlaps the top of the pendulum and exactly 6.4 mm (0.25 inches) will be held in the jaws.
2. Select an appropriate weight and a hole to give a reading between 2 and 6 on the scale.
   - NOTE:: The specimen should be brought to an approximate contact with the pendulum vane before applying force to avoid oscillation in the early stages.
3. Press the System Reset button. The display must read 00 - 000 - 00.
4. Press the Motor - Direction switch to cause the clamp arm to press the specimen against the pendulum.
5. Repeat step 4 in the opposite direction to establish both a left scale reading, a right scale reading, and an average reading.
6. Record the average scale reading.
7. Press the Select Button to attain the milligram calculation and record.
8. Repeat steps 1 through 7 for each specimen.

The following procedure should be used to obtain Gurley stiffness samples. A set of samples should be taken from five napkins. The longitudinal and transverse centerlines of each napkin is identified. The peel strip is removed and the garment adhesive is dusted with talc or corn starch. A sample 12.7 mm x 25.4 mm is cut from each napkin at the intersection of the two centerlines. Two additional samples of 12.7 mm x 25.4 mm are cut along the longitudinal centerline. The two additional samples are cut about 63.5 mm in front of and behind the first sample. The three samples from each napkin should be cut and handled carefully so as not to affect the sample stiffness. The Gurley stiffness is measured for each sample and the values are recorded as the stiffness of the central absorbent zone.

Next, one must identify parallel planes which are 63.5 mm in front of and behind the transverse centerline of the napkin. The width of the napkin is then measured at the narrowest point between the two planes so established. This is the width (C) of the napkin. The absorbent width is measured at the same point along the napkin. This is the width (B) of the absorbent. The width of the central absorbent zone is also measured at the same point along the napkin. This is the width (A) of the central absorbent zone. Points are then identified on the napkin which are located at the intersection of the parallel lines identified above and another set of lines which are parallel to the longitudinal centerline and at a distance of (C/4 + 6.4 mm) from the centerline. Take a 25.4 mm x 12.7 mm sample at each of these four points on the napkin. The Gurley stiffness of these samples is measured and are recorded as the stiffness of the adjacent zones.

Six additional points, three on each side of the longitudinal centerline, are identified which are about 7 mm from the longitudinal sides of the napkin. A sample 12.7 mm x 25.4 mm is cut from each of these points. The Gurley stiffness of each sample is measured and recorded as the "stiffness at the peripheral zone" of the napkin. The average for the stiffness of the peripheral zones is calculated and recorded as the "average stiffness of the peripheral zones" of the napkin. The average stiffness of the peripheral zones is then subtracted from the average stiffness of the central absorbent zone of the napkin and is recorded as the difference in stiffness between the central absorbent zone and the peripheral zones of the napkin.

The average of the values of the stiffness of the central absorbent zone is calculated and recorded as the "average stiffness of the central absorbent zone" for the napkin. The average of the values of the stiffness of the adjacent zones is calculated and recorded as the "average stiffness of the adjacent zones" for the napkin. The "average stiffness of the central absorbent zone" is then divided by the "average stiffness of the adjacent zones" and is reported as the ratio of the central absorbent zone to the adjacent zone stiffness.

The central absorbent zone 16 also has a ratio of machine direction stiffness to transverse direction stiffness of about 1.5 to 1.0, preferably about 1.8 to 1.0. Table 1 shows comparative data between the inventive product and two commercially available sanitary napkins which are relatively thin in caliper. The sanitary napkin also has a difference in stiffness between the central absorbent zone and peripheral zone of at least 150 milligrams. See Table 2 below. Lastly, the stiffness ratio of the central absorbent zone to the adjacent zones is greater than 2:1. See Table 3 below.

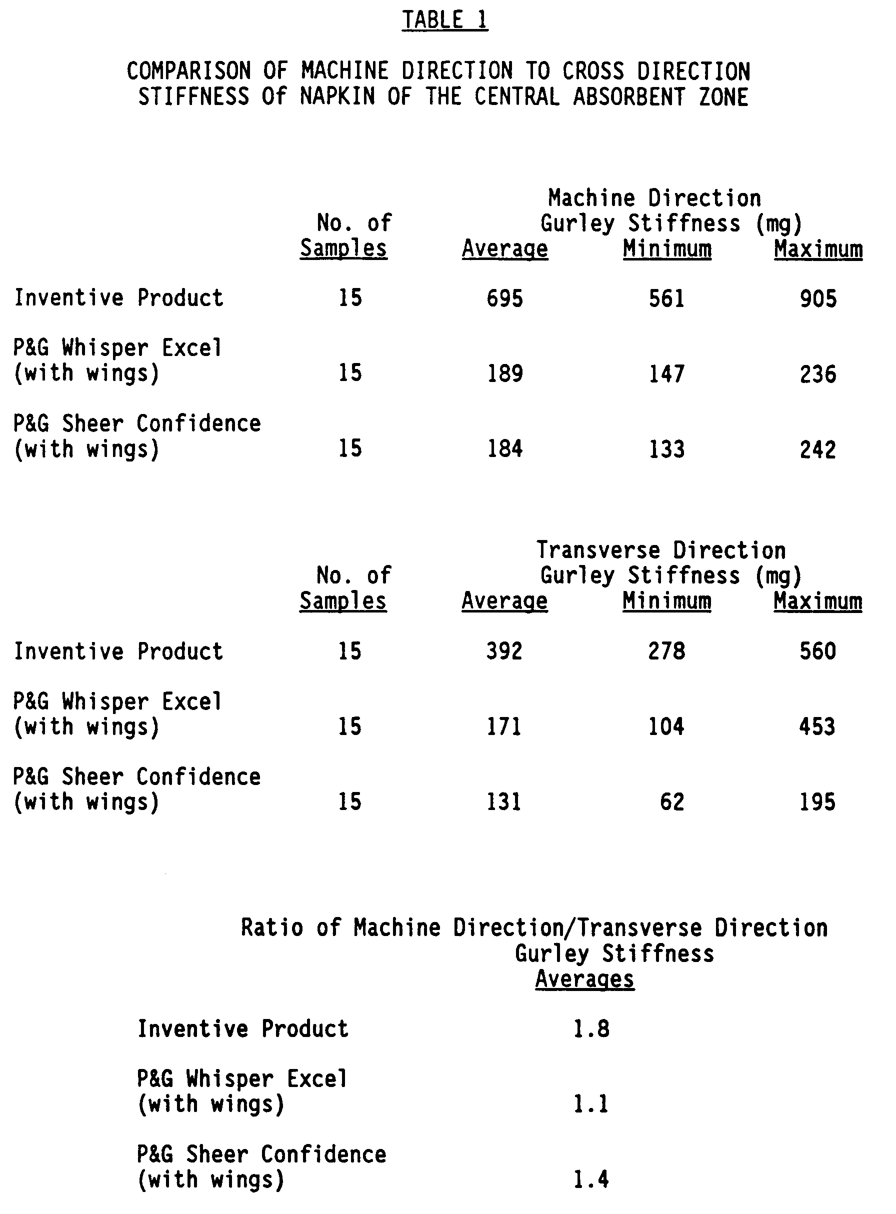

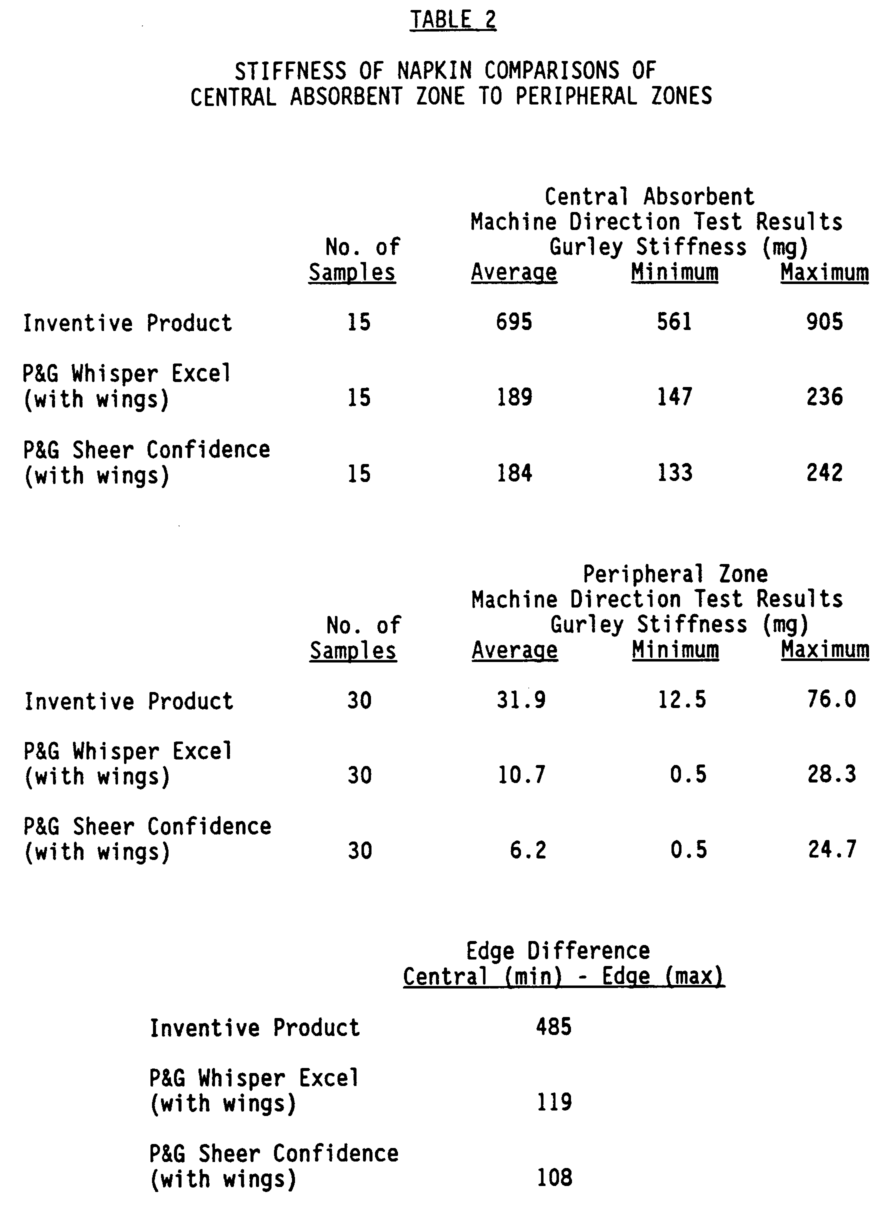

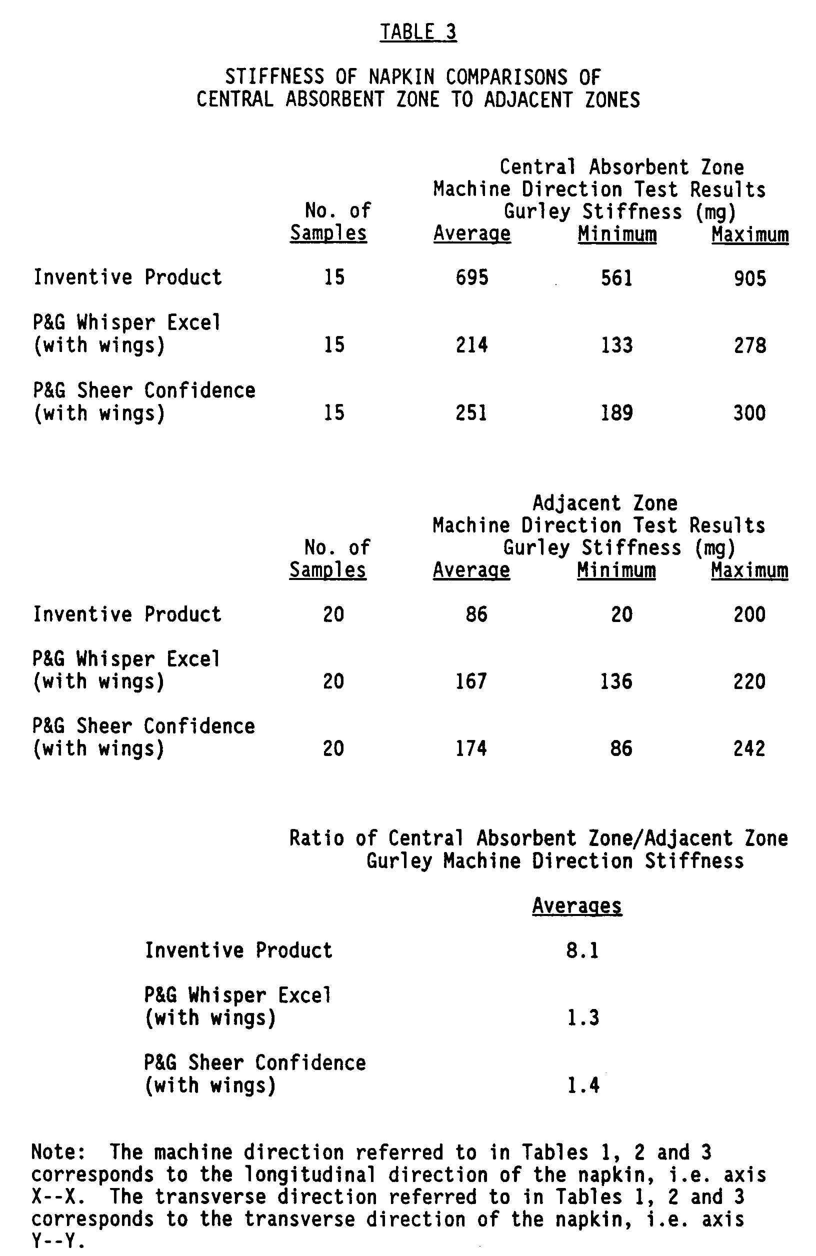

Referring to Fig. 2, the central absorbent zone 16 is depicted as being a composite comprised of a hydrophilic material 22 and a hydrocolloidal material 24. The hydrophilic material 22 can be various natural or synthetic fibers, including cellulose fibers, surfactant-treated meltblown fibers, wood pulp fibers, regenerated cellulose or cotton fibers, or a blend of pulp and other fibers. A preferred material is an airlaid tissue. The hydrocolloidal material 24, commonly referred to as a superabsorbent, can be a hydrogel-forming polymer composition which is water-insoluble, slightly cross-linked, and partially neutralized. It can be prepared from an unsaturated polymerizable, acid group-containing monomers and cross-linked agents. Such superabsorbents are taught in U.S. patents 4,798,603 issued to Meyers et al., Re. 32,649 issued to Brandt et al. and U.S. 4,467,012 issued to Pedersen et al., as well as in published European Patent Application 0,339,461 to Kellenberger.

Superabsorbents are very good at retaining body fluids. Superabsorbents have the ability to absorb a great amount of fluid in relation to their own weight. Typical superabsorbents used in sanitary napkins can absorb anywhere from 5 to 60 times their weight in blood. However, the absorption mechanism is not a rapid absorption and is usually slower than the rate of fluid absorption by the cellulose fluff material. The placement of the superabsorbent material in the center or lower portion of the napkin provides additional time for the superabsorbent to absorb the fluid from a transfer member.

It has been found that superabsorbents having a high mechanical stability in the swollen state, an ability to rapidly absorb fluid, and ones having a strong liquid binding capacity perform well in catamenial devices. Hydroxyfunctional polymers have been found to be good superabsorbents for this application. A hydrogel-forming polymer, specifically a partially neutralized cross-linked copolymer of polyacrylic acid and polyvinyl alcohol is preferred. After the polymer is formed, it is mixed with about a 1% anhydrous citric acid powder. The citric acid has been found to increase the ability of the superabsorbent to absorb menses and blood. This is particularly good for use in a sanitary napkin or feminine pad. The finely ground, anhydrous citric acid powder, which is void of water, along with trace amounts of fumed silica, is mixed with the polymer which has been screened to an appropriate particle size. This mixture can then be formed into a composite or a laminate structure. Such superabsorbents can be obtained from Dow Chemical, Hoechst-Celeanese, and Stockhausen, Inc., among others. This superabsorbent is a partially neutralized salt of cross-linked copolymer of polyacrylic acid and polyvinyl alcohol having an absorbency under load value above 25.

The superabsorbent 24 should have a high absorbency under load. That is, it should have the ability to expand or swell under a restraining pressure, typically about 0.3 psi*. The absorbency under load value is a function of gel strength, osmotic pressure within the gel and the composition of the polymer itself. The absorbency under load value also pertains to the ability of the gel to swell against other superabsorbent particles as well as against adjacent fibers when under pressure. For purposes of this invention, a superabsorbent having a high absorbency under load is defined as having a value of 20 or higher. A preferred absorbency under load value is 25 or higher. The test for determining an absorbency under load value is taught on page 7, lines 14-52 of published European Patent Application 0,339,461 (Kellenberger) and assigned to the present assignee.
* 1 psi = 0.069 bar

Referring to Figs. 3 and 4, the central absorbent zone 16 can also be constructed as a laminate 26 comprised of a hydrocolloidal material 24 positioned within a folded hydrophilic material such as a pulp layer 28. In Fig. 4, a laminate 30 is shown wherein two airlaid webs 32 and 34 enclose a superabsorbent 36 therebetween. The laminate 30 is retained in place upon the baffle 12 by a liquid-permeable cover 38. The cover 38 can be adhered about its outer periphery to the baffle 12. The cover 38 is designed to contact the body of the wearer and can be constructed of a woven or non-woven, natural or synthetic material which is easily penetrated by body fluid. Suitable materials include bonded carded webs of polyester, polypropylene, nylon, or other heat-bondable fibers. Other polyolefins such as copolymers of polypropylene and polyethylene, linear low-density polyethylene, finely-perforated film webs and net material also work well. A preferred material is a spunbonded polypropylene, non-apertured web which contains about 1 to 6% titanium dioxide pigment to give it a clean white appearance. A white uniform spunbonded material is desirable because the color exhibits good masking properties to hide menses which has passed through it and the material has sufficient strength after being perforated in the longitudinal direction to resist being torn. U.S. patents 4,801,494 issued to Datta et al. and 4,908 026 issued to Sukiennik et al. teach various cover materials which can be used with this sanitary napkin.

The liquid-permeable cover 38 can also contain a plurality of apertures 40 formed therein and the apertures 40 can be arranged along the longitudinal center line X--X, if desired. The apertures 40 will increase the rate at which body fluids can penetrate down into the absorbent laminate 30. The cover 38 can also be treated with a surfactant to make it more hydrophilic. The surfactant can include topical additions or internally applied materials like polysiloxanes.

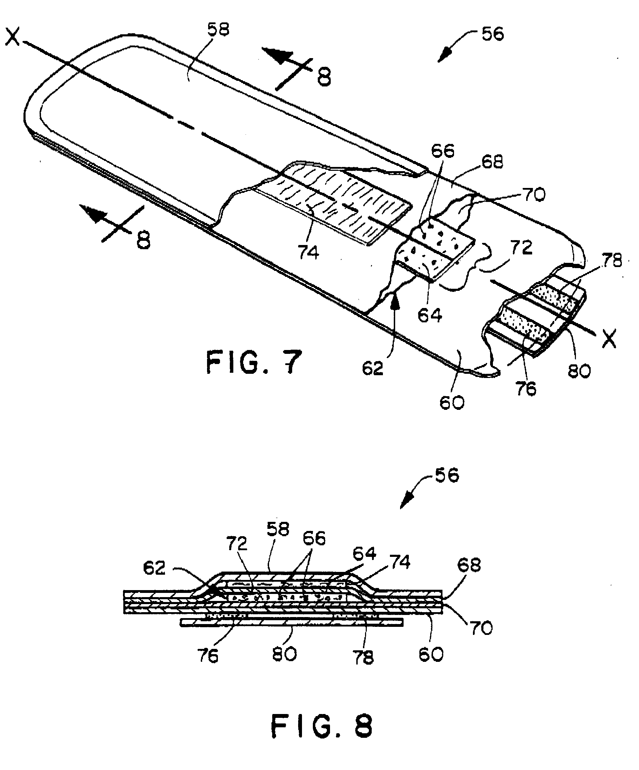

Referring to Figs. 5 and 6, two additional laminate embodiments are shown wherein a laminate is positioned between a baffle 12 and a cover 38. In Fig. 5, a laminate 42 is shown made up of a hydrocolloidal material 44 adhered to a layer of cellulose pulp 46. The hydrocolloidal material 44 can be adhered to both the baffle 12 and the pulp layer 46 by a construction adhesive 48. It should be noted that the pulp layer 46 can also be adhered to the cover 38 and/or the baffle 12 as well. In Fig. 6, a laminate 50 is shown consisting of a hydrocolloidal material 52 completely enclosed by a nonwoven web 54. The nonwoven web 54 can be a surfactant treated meltblown web 54 which is secured to the baffle 12 and/or the cover 38 to give the sanitary napkin integrity.

Referring to Figs. 7 and 8, a sanitary napkin 56 is shown having a liquid-permeable cover 58, a liquid-impermeable baffle 60 and an absorbent 62 enclosed therebetween. The absorbent 62 can be a composite or a laminate which includes a hydrophilic material 64 and a superabsorbent 66. The composite or laminate is sandwiched between two layers of tissue 68 and 70. The tissue layers 68 and 70 enclose and extend laterally outward from the composite or laminate and the combination forms the absorbent 62. The absorbent 62 has a central absorbent zone 72 which is aligned along the longitudinal central axis X--X of the sanitary napkin 56. The central absorbent zone 72 is similar in construction to the central absorbent zone 16, explained above.

Positioned above the absorbent 62 is a transfer member 74. The transfer member 74 is a nonwoven polypropylene meltblown web which facilitates movement of body fluid downward and outward from the cover 58 to distant areas of the central absorbent zone 72. Preferably, the transfer member 74 is aligned along the longitudinal axis X--X of the sanitary napkin 56 and is sized and configured to correspond to the shape and dimensions of the central absorbent zone 72. A description of a transfer layer is taught in U.S. 4,798,603 issued to Meyer et al and assigned to the present assignee. This patent is incorporated by reference and made a part hereof.

The sanitary napkin 56 also contains two longitudinally-extending strips of garment adhesive 76 and 78 which are attached to the exterior surface of the baffle 60. The garment adhesive is commercially available from National Starch Co. located at 10 Finderne Ave. Bridgewater, New Jersey 08807. The strips of garment adhesive 76 and 78 are used to secure the sanitary napkin 56 to the inside of the crotch portion of an undergarment so that it can be properly aligned with the vaginal opening. A peel strip 80 is releasably attached to the garment adhesive strips 76 and 78 and prevents the adhesive from becoming contaminated prior to attachment to the undergarment. The peel strip 80 can be a white Kraft paper coated on one side so that it can be released from a hot melt adhesive. The peel strip 80 is designed to be removed by the ultimate consumer just prior to placement of the sanitary napkin 56 in the undergarment.

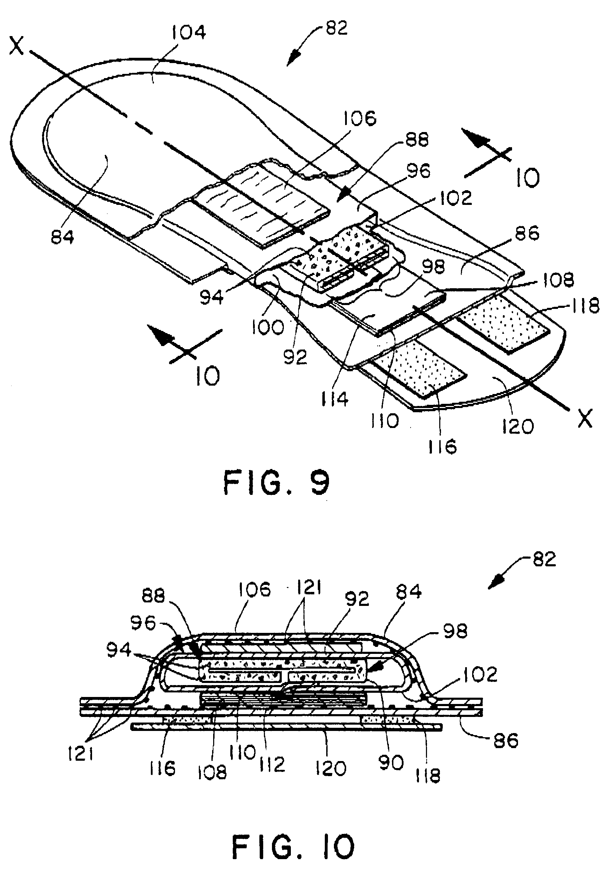

Referring to Figs. 9 and 10, another embodiment of a sanitary napkin 82 is shown having a racetrack profile with essentially parallel, longitudinally-extending sides which are connected by a smooth curve at each end. The sanitary napkin 82 is constructed of a liquid-permeable cover 84, a liquid-impermeable baffle 86 and an absorbent 88. The absorbent 88 is completely enclosed by both the cover 84 and the baffle 86 and is aligned along the longitudinal central axis X--X of the sanitary napkin 82. The absorbent 88 includes a laminate 90 constructed of a hydrophilic material 92 and a superabsorbent 94. Preferably, the hydrophilic material is C-folded and the superabsorbent 94 is contained therein. The absorbent 88 also includes a tissue layer 96 which is C-folded and overlapped upon itself. The tissue layer 96 extends laterally outward from the laminate 90 and is cut or formed into an hourglass profile, best seen in Fig. 9. The hourglass profile conveys a perception of comfort to the ultimate consumer.

The absorbent 88 has a central absorbent zone 98 and a pair of adjacent absorbent zones 100 and 102. The central absorbent zone 98 contains the superabsorbent 94 and represents the significant absorption portion of the sanitary napkin 82. The central absorbent zone is capable of absorbing a significant amount of body fluid, preferably at least about 20 grams, which is deposited onto the napkin 82. In Fig. 9, the central absorbent zone 98 is the area defined by the laminate 90. The adjacent zones 100 and 102 flank the central absorbent zone 98, but contain little if any superabsorbent. Preferably, the adjacent zones 100 and 102 do not contain superabsorbent. The adjacent zones 100 and 102 contain only the cover 84, the baffle 86 and the tissue layer 96. Each adjacent zone, 100 and 102, can absorb only a couple of grams of fluid but provides a lateral surface which prevents side leakage. All of the zones 98, 100 and 102 extend longitudinally at least 50% of the length of the napkin 82 and are aligned approximately parallel to the central axis X--X. Preferably, the three zones 98, 100 and 102 extend approximately the entire length of the napkin 82 and terminate at a peripheral seal line 104.

A transfer member 106 is positioned between the cover 84 and the absorbent 88 and is aligned along the longitudinal central axis of the napkin 82. The transfer member 106, as described above, can vary in size and configuration, but preferably will have a length and width which corresponds to the size of the central absorbent zone 98. The transfer member 106 is positioned vertically above the central absorbent zone 98 and facilitates movement of body fluid downward and outward from the cover 84 to distant areas of the absorbent 88.

A pulp layer 108 is positioned between the absorbent 88 and the baffle 86 and is aligned along the longitudinal central axis X--X of the napkin 82. The pulp layer 108 can be formed from a web of wood pulp fibers, such as common blotter paper and can contain a superabsorbent. The pulp layer 108 has relatively flat, first and second major surfaces, 110 and 112 respectively. One or both of the surfaces 110 and 112 can have a plurality of score lines 114 formed therein which extend parallel to the central longitudinal axis X--X of the napkin 82. The score lines 114 assist in wicking fluid lengthwise along the pulp layer 108. The pulp layer 108 preferably has a length and width corresponding to the central absorbent zone 98 and is vertically positioned below it. The pulp layer 108 facilitates movement of body fluid lengthwise to distant areas of the central absorbent zone 98. The pulp layer 108 also provides added stiffness to the central absorbent zone 98 and permits it to resist deformation when the napkin 82 is squeezed in the transverse direction. By resisting deformation, the central absorbent zone 98 is able to lay flat against the body and, therefore, will be in a much better position to absorb the body fluid discharged from the vaginal cavity.

The sanitary napkin 82 also has two strips of garment adhesive 116 and 118 secured to an exterior surface of the baffle 86. The garment adhesive provides a means for attaching the sanitary napkin to an inside surface of an undergarment. It should be noted that although two strips of garment adhesive 116 and 118 are shown, it is possible to use a single strip of adhesive or several smaller strips if desired. For best results, the sanitary napkin should have between 10 to 20 square inches of garment adhesive applied to the baffle 86. A releasable peel strip 120 is positioned over the two strips of garment adhesive 116 and 118 and serves to prevent the adhesive from becoming contaminated prior to use.

The sanitary napkin 82 also contains several locations which have been coated with a construction adhesive 121. The construction adhesive 121 serves to hold the various layers together and gives the napkin 82 integrity. Preferably, the sanitary napkin 82 will contain construction adhesive 121 to retain the cover 84 to both the baffle 86 and to the transfer member 106. The construction adhesive can also secure the pulp layer 108 to the baffle 86 and secure the laminate 90 to the tissue 96. It should be noted that the amount and location of the construction adhesive 121 can vary depending upon the size and shape of the product and the type of machine the product is manufactured on. The adhesive strength will also determine how much adhesive 121 is needed.

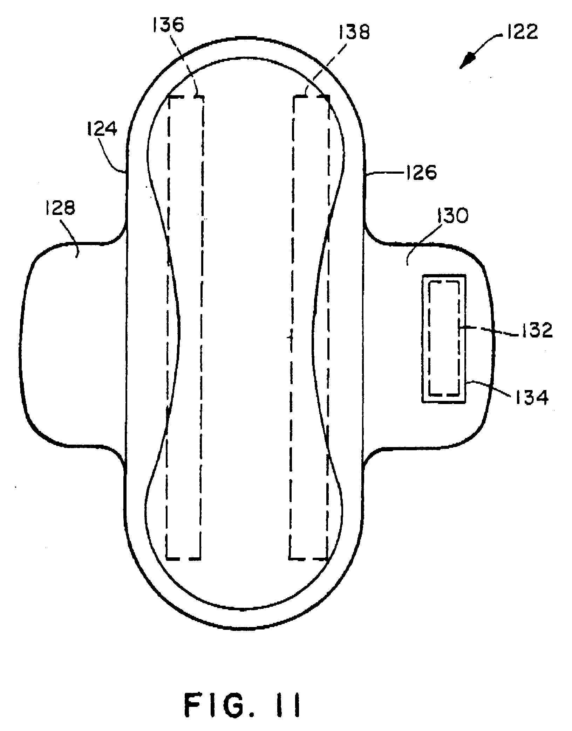

Referring to Fig. 11, a sanitary napkin 122 is shown which is similar in construction to the napkin 82 depicted in Fig. 9. The napkin 122 differs in that attached to its longitudinal sides 124 and 126 are a pair of wings 128 and 130. The wings 128 and 130 extend laterally outward from the napkin 122 and are designed to wrap around the outer surface of the crotch portion of an undergarment. At least one of the wings 128 and 130 has an adhesive strip 132 attached to its upper surface which is covered by a releasable peel strip 134. The sanitary napkin 122 also has two spaced-apart garment adhesive strips 136 and 138 secured to the exterior surface of the baffle. The garment adhesive strips 136 and 138 can be covered by a releasable peel strip (not shown). When the consumer is ready to wear the product, she removes the peel strip from the bottom of the napkin 122 and adheres the napkin 122 via the garment adhesive strips 136 and 138 to the interior surface of her undergarment. She then removes the peel strip 134 and wraps the wings 128 and 130 around the exterior surface of the undergarment and adheres one wing to the other wing via the adhesive 132.

### METHOD

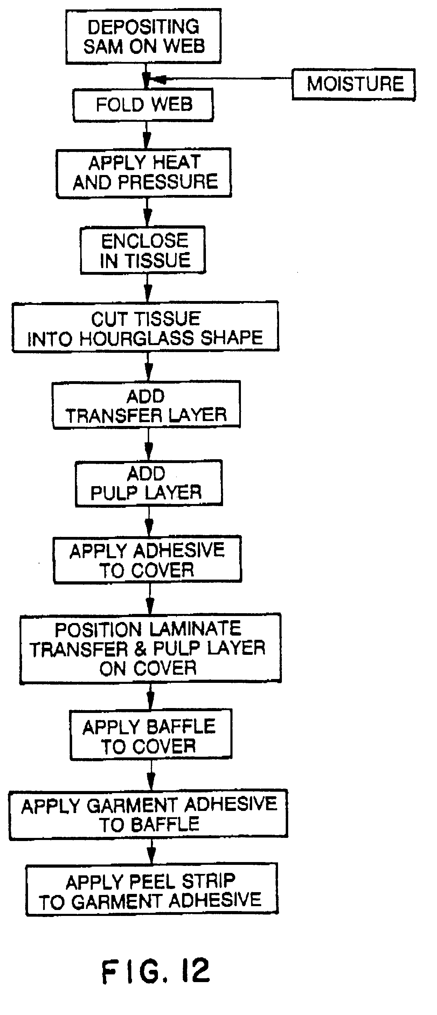

Referring to Fig. 12, a flow diagram is depicted showing a method of manufacturing the sanitary napkin of this invention. The method includes the steps of depositing a superabsorbent onto a web of airlaid tissue. Moisture in the form of water particles or steam can be added to the airlaid tissue. The moisture causes the superabsorbent to acquire a tacky surface. Other ways to obtain a tacky surface is to treat the airlaid tissue with a hot melt pressure-sensitive adhesive, a latex binder or poly-vinyl-alcohol. The tissue is then folded about the superabsorbent to form a laminate. Conventional C-folds can be easily formed with commercially available equipment. The tacky surface on the superabsorbent aids in retaining the shape of the laminate, but it is beneficial to subject the laminate to both heat and pressure to obtain a predetermined shape. This can be accomplished by running the laminate between the nip of two rollers. The heat serves to drive off excess moisture and helps retain the fold formed in the web. The laminate is then enclosed in a tissue layer or, alternatively, it can be sandwiched between two tissue layers. The tissue layer or layers have a larger surface area than the laminate and therefore overlaps the edges of the laminate. Preferably, the tissue layer or layers are cut into an hourglass profile. The hourglass shape conveys a perception of comfort to the consumer since it is narrow at the crotch portion and allows the napkin to fit between the thighs.

The method further includes the steps of positioning the hourglass profile between a transfer member and a pulp layer to form an absorbent structure. An adhesive is applied to a major surface of a liquid-permeable cover and the absorbent structure is adhered to the adhesive. It should be noted that the absorbent structure is aligned along the central longitudinal axis of the cover. A liquid-impermeable baffle is then placed over the absorbent structure and it, too, is adhered to the cover. For best results, the baffle and the cover should have coterminous edges. Preferably, the baffle and the cover have a racetrack profile with a pair of longitudinally-extending sides. The racetrack shape conveys to the consumer a perception that the napkin has sufficient width to prevent side leakage. A garment adhesive is then applied to the exterior surface of the baffle and a releasable peel strip is positioned over the garment adhesive. The sanitary napkin is then ready to be packaged and sold.

## Claims

1. A sanitary napkin (10,56,82) having a longitudinal axis, comprising:
a. a liquid-permeable cover (38,58,84);
b. a liquid-impermeable baffle (12,60;86), and
c. an absorbent (14,62,88) being enclosed between said cover (38,58,84) and said baffle (12,60,86), said absorbent having a longitudinally-extending central absorbent zone (16;72,98) aligned along the longitudinal axis (x-x) of said napkin (10,56,82),
characterized by
said central absorbent zone (16;72,98) having a width of less than 5.08 cm (2 inches) and less than the width of said absorbent (14,62,88) measured approximate said central transverse axis of said napkin (10,56,82) and being capable of absorbing at least 20 grams of the body fluid deposited on said napkin (10,56,82),
said central absorbent zone having a stiffness value of at least 150 mg greater than the stiffness value of adjacent portions (100,102) to said central absorbent zone when measured by subtracting a minimum machine direction Gurley stiffness value of said central absorbent zone from a maximum machine direction Gurley stiffness value of said adjacent portions (100,102) and/or said central absorbent zone having a stiffness ratio of about 2:1 compared to said adjacent portions (100,102) when measured by dividing an average machine direction Gurley stiffness value of said central absorbent zone by an average machine direction Gurley stiffness value of said adjacent portions (100,102) and said napkin (10,56,82) having a caliper of less than about 5 mm.

2. The sanitary napkin of claim 1 wherein said central absorbent zone (16;72,98) has a width which is less than 75% of the width of said absorbent (14,62,88) measured approximate said central transverse axis of said napkin (10,56,82) and less than 60% of the width of said napkin, when measured across the narrowest portion of said napkin.

3. A sanitary napkin (10,56,82) having a longitudinal axis, comprising:
a. a liquid-permeable cover (38,58,84);
b. a liquid-impermeable baffle (12,60;86), and
c. an absorbent (14,62,88) being enclosed between said cover (38,58,84) and said baffle (12,60,86), said absorbent having a longitudinally-extending central absorbent zone (16;72,98) aligned along the longitudinal axis (x-x) of said napkin (10,56,82),
characterized by
said central absorbent zone (16;72,98) having a width which is less than 75% of the width of said absorbent (14,62,88) measured approximate said central transverse axis of said napkin (10,56,82) and less than 60% of the width of said napkin when measured across the narrowest portion of said napkin, and being capable of absorbing at least 20 grams of the body fluid deposited on said napkin (10,56,82),
said central absorbent zone having a stiffness value of at least 150 mg greater than the stiffness value of adjacent portions (100,102) to said central absorbent zone when measured by subtracting a minimum machine direction Gurley stiffness value of said central absorbent zone from a maximum machine direction Gurley stiffness value of said adjacent portions (100,102) and/or said central absorbent zone having a stiffness ratio of about 2:1 compared to said adjacent portions (100,102) when measured by dividing an average machine direction Gurley stiffness value of said central absorbent zone by an average machine direction Gurley stiffness value of said adjacent portions (100,102) and said napkin (10,56,82) having a caliper of less than about 5 mm.

4. The sanitary napkin of one of claims 1 to 3 wherein said central absorbent zone (16,72,98) has a Gurley stiffness of at least 500 milligrams.

5. The sanitary napkin of one of claims 1 to 4 wherein said central absorbent zone (16) is a laminate (50) comprised of a superabsorbent (52) enclosed by a nonwoven, preferably an airlaid web (54).

6. The sanitary napkin of one of claims 1 to 5 wherein said central absorbent zone is comprised of a superabsorbent (52) enclosed by an airlaid web (54).

7. The sanitary napkin of one of claims 1 to 5 wherein said central absorbent zone (16) is a laminate (30) comprised of a superabsorbent (36) positioned between two airlaid webs (32,34).

8. The sanitary napkin of one of the preceding claims wherein said central absorbent zone (16) is a composite (14) comprised of hydrophilic fibers (22) and a superabsorbent (24).

9. The sanitary napkin of one of the preceding claims wherein said central absorbent zone comprises a layer of superabsorbent adhered to a layer of cellulose pulp.

10. The sanitary napkin of one of claims 5 to 9 wherein said superabsorbent is treated with citric acid to increase its affinity to absorb menses.

11. The sanitary napkin of one of claims 5 to 10 wherein said superabsorbent is a copolymer of polyacrylic acid and polyvinyl alcohol.

12. The sanitary napkin of one of claims 5 to 10 wherein said superabsorbent is a hydroxyfunctional polymer.

13. The sanitary napkin of one of claims 5 to 12 wherein said superabsorbent has an absorbency under load value of at least about 20.

14. The sanitary napkin of one of the preceding claims wherein said central absorbent zone (16,72,98) has a width of between 1.27 cm and 5.08 cm. (0.5 and 2.0 inches), said zone extending approximately the entire length of said napkin, and said napkin having a caliper of less than about 4 mm.

15. The sanitary napkin of one of the preceding claims wherein said central absorbent zone (16) is a composite comprised of meltblown fibers (22) and a superabsorbent (24).

16. The sanitary napkin of claim 15 wherein said central absorbent (16) zone is a laminate (50) comprised of a hydrocolloid material (52) enclosed in a hydrophilic material (54).

17. The sanitary napkin of claim 16 wherein said hydrophilic material is an airlaid tissue.

18. The sanitary napkin of claim 16 or 17 wherein said hydrocolloid material has an absorbency under load value of at least about 20.

19. The sanitary napkin of one of the preceding claims wherein said absorbent (88) is in contact with a layer of tissue (96) which has an hourglass profile.

20. The sanitary napkin of one of the preceding claims wherein the exterior periphery of said napkin has a racetrack profile.

21. The sanitary napkin of one of the preceding claims wherein said central absorbent zone (98) is flanked by a pair of adjacent zones (100,102) and said central zone is capable of absorbing at least 80%, preferably about 90% of the body fluid deposited onto said napkin.

22. The sanitary napkin of one of the preceding claims wherein said absorbent (16) includes a layer of superabsorbent (44) and a layer of pulp (46) sandwiched between said cover (38) and said baffle (12).

23. The sanitary napkin of one of the preceding claims wherein said absorbent includes a layer of superabsorbent (44) bonded to said baffle (12) and a fibrous layer (46) secured to both the said superabsorbent (44) and to said cover (38).

24. The sanitary napkin of one of the preceding claims wherein said central absorbent zone (98) has a caliper greater than the caliper of said adjacent zones.

25. The sanitary napkin of one of the preceding claims wherein said central absorbent zone (98) can absorb about 90% of the body fluid deposited onto said napkin.

26. The sanitary napkin of one of the preceding claims wherein said central absorbent zone (98) contains a hydrocolloid material and said adjacent zones are void of any hydrocolloid material.

27. The sanitary napkin of one of the preceding claims wherein said absorbent is a laminate comprised of a superabsorbent material sandwiched between two layers of airlaid tissue.

28. The sanitary napkin of one of the preceding claims wherein said superabsorbent is a copolymer of polyacrylic acid and polyvinyl alcohol.

29. The sanitary napkin of one of the preceding claims, wherein a transfer member (74,106) is positioned between said cover (58,84) and said absorbent (62,88) and is aligned along the longitudinal axis (x-x) of said napkin, said transfer member (74,106) facilitating movement of body fluid downward and outward to distant areas of said absorbent.

30. The sanitary napkin of one of the preceding claims wherein a pulp layer (108) is positioned between said absorbent (14,63,88) and said baffle (12,60,86) and is preferably aligned along the longitudinal axis (x-x) of said napkin, said pulp layer (108) providing added stiffness to said central absorbent zone to resist deformation when said napkin is squeezed in the transverse direction.

31. The sanitary napkin of claim 30 wherein said pulp layer (108) is scored in the longitudinal direction.

32. The sanitary napkin of claim 30 or 31 wherein said pulp layer (108) has first and second major surfaces (110,112) and both surfaces are scored.

33. The sanitary napkin of one of the preceding claims wherein said absorbent (88) has a width less than the width of said napkin and contains a superabsorbent.

34. The sanitary napkin according to one of the preceding claims, further comprising:
d. at least one tissue layer (96) enclosing and extending laterally outward from said absorbent,
e. a transfer member (106) positioned between said cover (84) and said absorbent (88) and aligned along the longitudinal central axis of said napkin, said transfer member (106) facilitating movement of body fluid downward and outward from said cover to distant areas of said absorbent, and
f. a pulp layer (108) positioned between said absorbent (88) and said baffle (86) and aligned along the longitudinal axis of said napkin, said pulp layer (108) providing added stiffness to said absorbent to resist deformation when said napkin is squeezed in the transverse direction.

35. The sanitary napkin according to one of the preceding claims wherein:
said liquid-permeable cover (84) has a pair of longitudinal sides, and further comprising
d. at least one tissue layer (96) enclosing and extending laterally outward from said absorbent, said tissue layer having an hourglass profile,
e. a transfer member (106) positioned between said cover (84) and said absorbent (88) and aligned along the longitudinal central axis of said napkin, said transfer member (106) facilitating movement of body fluid downward and outward from said cover to distant areas of said absorbent;
f. a pulp layer (108) positioned between said absorbent (88) and said baffle (86) and aligned along the longitudinal axis of said napkin, said pulp layer providing added stiffness to said absorbent,
g. adhesive means (121) for securing said cover to said transfer member, tissue layer and baffle for securing said baffle to said pulp layer, and for securing said tissue layer to said absorbent and optionally,
h. a garment adhesive (116,118) attached to said baffle (86) for securing said napkin to the crotch portion of an undergarment, and
i. a peel strip (120) releasably attached to said garment adhesive (116,118), for protecting said garment adhesive from becoming contaminated prior to attachment to said undergarment.

36. The sanitary napkin of claim 35 wherein said transfer member (106), said absorbent (88) and said pulp layer (108) are vertically aligned relative to each other and each has approximately the same width with coterminous longitudinally-extending sides, said transfer member, absorbent and pulp layer forming a central absorbent zone capable of absorbing at least about 90% of the body fluid deposited onto said sanitary napkin.

37. The sanitary napkin of one of claims 3 to 36 wherein the central absorbent zone has a width of less than 5.08 cm (2 inches).

## Patentansprüche

1. Damenbinde (10, 56, 82) mit einer Längsachse, mit:
a. einer flüssigkeitsdurchlässigen Abdeckung (38, 58, 84);
b. einer flüssigkeitsundurchlässigen Schutzschicht (12, 60; 86), und
c. einem zwischen der Abdeckung (38, 58, 84) und der Schutzschicht (12, 60; 86) eingeschlossenen Absorbens (14, 62, 88), wobei das Absorbens eine längs verlaufende zentrale saugfähige Zone (16; 72, 98) aufweist, welche entlang der Längsachse (x-x) der Binde (10, 56, 82) ausgerichtet ist,
dadurch gekennzeichnet, daß
die zentrale saugfähige Zone (16; 72, 98) eine Breite von weniger als 5,08 cm (2 Inch) und weniger als der Breite des Absorbens (14, 62, 88), gemessen nahe der Mittelquerachse der Binde (10, 56, 82), aufweist, und mindestens 20 Gramm des auf der Binde (10, 56, 82) abgelagerten Körperfluids absorbieren kann,
wobei die zentrale saugfähige Zone einen Steifheitswert von mindestens 150 mg mehr als der Steifheitswert von zu der zentralen saugfähigen Zone benachbarten Bereichen (100, 102) aufweist, wenn durch Subtraktion eines minimalen Gurley-Steifheitswerts in Maschinenrichtung der zentralen saugfähigen Zone von einem maximalen Gurley-Steifheitswert in Maschinenrichtung der benachbarten Bereiche (100, 102) gemessen, und/oder die zentrale saugfähige Zone ein Steifheitsverhältnis von etwa 2:1 im Vergleich zu den benachbarten Bereichen (100, 102) aufweist, wenn durch Division eines durchschnittlichen Gurley-Steifheitswerts in Maschinenrichtung der zentralen saugfähigen Zone durch einen durchschnittlichen Gurley-Steifheitswert in Maschinenrichtung der benachbarten Bereiche (100, 102) gemessen, und die Binde (10, 56, 82) eine Dicke von weniger als etwa 5 mm aufweist.

2. Damenbinde gemäß Anspruch 1, bei der die zentrale saugfähige Zone (16; 72, 98) eine Breite aufweist, welche nahe der Mittelquerachse der Binde (10, 56, 82) gemessen weniger als 75% der Breite des Absorbens (14, 62, 88) beträgt, und über den schmälsten Bereich der Binde gemessen weniger als 60% der Breite der Binde beträgt.

3. Damenbinde (10, 56, 82) mit einer Längsachse, mit:
a. einer flüssigkeitsdurchlässigen Abdeckung (38, 58, 84);
b. einer flüssigkeitsundurchlässigen Schutzschicht (12, 60; 86), und
c. einem zwischen der Abdeckung (38, 58, 84) und der Schutzschicht (12, 60; 86) eingeschlossenen Absorbens (14, 62, 88), wobei das Absorbens eine längs verlaufende zentrale saugfähige Zone (16; 72, 98) aufweist, welche entlang der Längsachse (x-x) der Binde (10, 56, 82) ausgerichtet ist,
dadurch gekennzeichnet, daß
die zentrale saugfähige Zone (16; 72, 98) eine Breite aufweist, welche nahe der Mittelquerachse der Binde (10, 56, 82) gemessen weniger als 75% der Breite des Absorbens (14, 62, 88) beträgt, und über den schmälsten Bereich der Binde gemessen weniger als 60% der Breite der Binde beträgt, und mindestens 20 Gramm des auf der Binde (10, 56, 82) abgelagerten Körperfluids absorbieren kann,
wobei die zentrale saugfähige Zone einen Steifheitswert von mindestens 150 mg mehr als der Steifheitswert von zu der zentralen saugfähigen Zone benachbarten Bereichen (100, 102) aufweist, wenn durch Subtraktion eines minimalen Gurley-Steifheitswerts in Maschinenrichtung der zentralen saugfähigen Zone von einem maximalen Gurley-Steifheitswert in Maschinenrichtung der benachbarten Bereiche (100, 102) gemessen, und/oder die zentrale saugfähige Zone ein Steifheitsverhältnis von etwa 2:1 im Vergleich zu den benachbarten Bereichen (100, 102) aufweist, wenn durch Division eines durchschnittlichen Gurley-Steifheitswerts in Maschinenrichtung der zentralen saugfähigen Zone durch einen durchschnittlichen Gurley-Steifheitswert in Maschinenrichtung der benachbarten Bereiche (100, 102) gemessen, und die Binde (10, 56, 82) eine Dicke von weniger als etwa 5 mm aufweist.

4. Damenbinde gemäß einem der Ansprüche 1 bis 3, bei der die zentrale saugfähige Zone (16; 72, 98) eine Gurley-Steifheit von mindestens 500 Milligramm aufweist.

5. Damenbinde gemäß einem der Ansprüche 1 bis 4, bei der die zentrale saugfähige Zone (16) ein Laminat (50) ist, welches aus einem Superabsorptionsmittel (52), welches von einem Vlies, vorzugsweise einer luftabgelegten Bahn (54), eingeschlossen ist, zusammengesetzt ist.

6. Damenbinde gemäß einem der Ansprüche 1 bis 5, bei der die zentrale saugfähige Zone aus einem Superabsorptionsmittel (52), welches von einer luftabgelegten Bahn (54) eingeschlossen ist, zusammengesetzt ist.

7. Damenbinde gemäß einem der Ansprüche 1 bis 5, bei der die zentrale saugfähige Zone (16) ein Laminat (30) ist, welches aus einem Superabsorptionsmittel (36), welches zwischen zwei luftabgelegten Bahnen (32, 34) positioniert ist, zusammengesetzt ist.

8. Damenbinde gemäß einem der vorhergehenden Ansprüche, bei der die zentrale saugfähige Zone (16) ein Verbundstoff (14) ist, welcher aus hydrophilen Fasern (22) und einem Superabsorptionsmittel (24) zusammengesetzt ist.

9. Damenbinde gemäß einem der vorhergehenden Ansprüche, bei der die zentrale saugfähige Zone eine Schicht Superabsorptionsmittel aufweist, die an einer Schicht Zellulosezellstoff haftet.

10. Damenbinde gemäß einem der Ansprüche 5 bis 9, bei der das Superabsorptionsmittel zur Erhöhung seiner Neigung zur Absorption von Menses mit Zitronensäure behandelt ist.

11. Damenbinde gemäß einem der Ansprüche 5 bis 10, bei der das Superabsorptionsmittel ein Copolymer von Polyacrylsäure und Polyvinylalkohol ist.

12. Damenbinde gemäß einem der Ansprüche 5 bis 10, bei der das Superabsorptionsmittel ein hydroxyfunktionelles Polymer ist.

13. Damenbinde gemäß einem der Ansprüche 5 bis 12, bei der das Superabsorptionsmittel einen Wert des Absorptionsvermögens unter Belastung von mindestens etwa 20 aufweist.

14. Damenbinde gemäß einem der vorhergehenden Ansprüche, bei der die zentrale saugfähige Zone (16; 72, 98) eine Breite zwischen 1,27 cm und 5,08 cm (0,5 und 2,0 Inch) aufweist, wobei sich die Zone ungefähr die gesamte Länge der Binde erstreckt, und die Binde eine Dicke von weniger als etwa 4 mm aufweist.

15. Damenbinde gemäß einem der vorhergehenden Ansprüche, bei der die zentrale saugfähige Zone (16) ein Verbundstoff ist, welcher aus schmelzgeblasenen Fasern (22) und einem Superabsorptionsmittel (24) zusammengesetzt ist.

16. Damenbinde gemäß Anspruch 15, bei der die zentrale saugfähige Zone (16) ein Laminat (50) ist, das aus einem Hydrokolloidmaterial (52), welches in einem hydrophilen Material (54) eingeschlossen ist, zusammengesetzt ist.

17. Damenbinde gemäß Anspruch 16, bei der das hydrophile Material luftabgelegtes Tissue ist.

18. Damenbinde gemäß Anspruch 16 oder 17, bei der das Hydrokolloidmaterial einen Wert des Absorptionsvermögens unter Belastung von mindestens etwa 20 aufweist.

19. Damenbinde gemäß einem der vorhergehenden Ansprüche, bei der das Absorbens (88) mit einer Tissueschicht (96) mit einem Sanduhrprofil in Kontakt ist.

20. Damenbinde gemäß einem der vorhergehenden Ansprüche, bei der die äußerere Peripherie der Binde ein Rennbahnprofil aufweist.

21. Damenbinde gemäß einem der vorhergehenden Ansprüche, bei der ein Paar benachbarter Zonen (100, 102) seitlich von der zentralen saugfähigen Zone (98) liegen, und die zentrale Zone mindestens 80%, vorzugsweise etwa 90% des auf der Binde abgelagerten Fluids absorbieren kann.

22. Damenbinde gemäß einem der vorhergehenden Ansprüche, bei der das Absorbens (16) eine Schicht Superabsorptionsmittel (44) und eine Schicht Zellstoff (46), welche zwischen der Abdeckung (38) und der Schutzschicht (12) liegen, aufweist.

23. Damenbinde gemäß einem der vorhergehenden Ansprüche, bei der das Absorbens eine Schicht Superabsorptionsmittel (44), welche an die Schutzschicht (12) gebunden ist, und eine Faserschicht (46), welche sowohl an dem Superabsorptionsmittel (44) als auch der Abdeckung (38) befestigt ist, aufweist.

24. Damenbinde gemäß einem der vorhergehenden Ansprüche, bei der die zentrale saugfähige Zone (98) eine Dicke aufweist, welche größer als die Dicke der benachbarten Zonen ist.

25. Damenbinde gemäß einem der vorhergehenden Ansprüche, bei der die zentrale saugfähige Zone (98) etwa 90% des auf der Binde abgelagerten Körperfluids absorbieren kann.

26. Damenbinde gemäß einem der vorhergehenden Ansprüche, bei der die zentrale saugfähige Zone (98) Hydrokolloidmaterial enthält und die benachbarten Zonen frei von jeglichem Hydrokolloidmaterial sind.

27. Damenbinde gemäß einem der vorhergehenden Ansprüche, bei der das Absorbens ein Laminat ist, das aus einem Superabsorptionsmaterial zusammengesetzt ist, welches zwischen zwei Schichten luftabgelegten Tissues liegt.

28. Damenbinde gemäß einem der vorhergehenden Ansprüche, bei der das Superabsorptionsmittel ein Copolymer von Polyacrylsäure und Polyvinylalkohol ist.

29. Damenbinde gemäß einem der vorhergehenden Ansprüche, bei der ein Übertragungselement (74, 106) zwischen der Abdeckung (58, 84) und dem Absorbens (62, 88) angeordnet ist und entlang der Längsachse (x-x) der Binde ausgerichtet ist, wobei das Übertragungselement (74, 106) die Bewegung von Körperfluid nach unten und nach außen zu entfernten Bereichen des Absorbens erleichtert.

30. Damenbinde gemäß einem der vorhergehenden Ansprüche, bei der eine Zellstoffschicht (108) zwischen dem Absorbens (14, 62, 88) und der Schutzschicht (12, 60; 86) liegt und vorzugsweise entlang der Längsachse (x-x) der Binde ausgerichtet ist, wobei die Zellstoffschicht (108) der zentralen saugfähigen Zone zusätzliche Steifheit verleiht, um einer Deformation zu widerstehen, wenn die Binde in Querrichtung zusammengedrückt wird.

31. Damenbinde gemäß Anspruch 30, bei der die Zellstoffschicht (108) in Längsrichtung mit Rillen versehen ist.

32. Damenbinde gemäß Anspruch 30 oder 31, bei der die Zellstoffschicht (108) eine erste und eine zweite Hauptoberfläche (110, 112) aufweist und beide Oberflächen mit Rillen versehen sind.

33. Damenbinde gemäß einem der vorhergehenden Ansprüche, bei der das Absorbens (88) eine Breite von weniger als der Breite der Binde aufweist und ein Superabsorptionsmittel enthält.

34. Damenbinde gemäß einem der vorhergehenden Ansprüche, die des weiteren folgendes umfaßt:
d. mindestens eine Tissueschicht (96), die das Absorbens umschließt und sich seitwärts von dem Absorbens nach außen erstreckt,
e. ein Übertragungselement (106), das zwischen der Abdeckung (84) und dem Absorbens (88) angeordnet ist und entlang der Längsmittelachse der Binde ausgerichtet ist, wobei das Übertragungselement (106) die Bewegung von Körperfluid nach unten und nach außen von der Abdeckung zu entfernten Bereichen des Absorbens erleichtert; und
f. eine Zellstoffschicht (108), die zwischen dem Absorbens (88) und der Schutzschicht (86) liegt und entlang der Längsachse der Binde ausgerichtet ist, wobei die Zellstoffschicht (108) dem Absorbens zusätzliche Steifheit verleiht, um einer Deformation zu widerstehen, wenn die Binde in Querrichtung zusammengedrückt wird.

35. Damenbinde gemäß einem der vorhergehenden Ansprüche, bei der:
die flüssigkeitsdurchlässige Abdeckung (84) ein Paar Längsseiten aufweist, des weiteren umfassend:
d. mindestens eine Tissueschicht (96), die das Absorbens umschließt und sich seitwärts von dem Absorbens nach außen erstreckt, wobei die Tissueschicht ein Sanduhrprofil aufweist,
e. ein Übertragungselement (106), das zwischen der Abdeckung (84) und dem Absorbens (88) angeordnet ist und entlang der Längsmittelachse der Binde ausgerichtet ist, wobei das Übertragungselement (106) die Bewegung von Körperfluid nach unten und nach außen von der Abdeckung zu entfernten Bereichen des Absorbens erleichtert;
f. eine Zellstoffschicht (108), die zwischen dem Absorbens (88) und der Schutzschicht (86) liegt und entlang der Längsachse der Binde ausgerichtet ist, wobei die Zellstoffschicht dem Absorbens zusätzliche Steifheit verleiht;
g. Haftmittel (121) zur Befestigung der Abdeckung an dem Übertragungselement, der Tissueschicht und der Schutzschicht, zur Befestigung der Schutzschicht an der Zellstoffschicht und zur Befestigung der Tissueschicht an dem Absorbens und wahlweise
h. ein an der Schutzschicht (86) angebrachtes Bekleidungshaftmittel (116, 118) zur Befestigung der Binde an dem Schrittbereich einer Unterwäsche, und
i. einen lösbar an dem Bekleidungshaftmittel (116, 118) angebrachten Abziehstreifen (120) zum Schutz des Bekleidungshaftmittels vor Verschmutzung vor dem Anbringen an der Unterwäsche.

36. Damenbinde gemäß Anspruch 35, bei der das Übertragungselement (106), das Absorbens (88) und die Zellstoffschicht (108) relativ zueiander vertikal ausgerichtet sind und jede dieser Lagen ungefähr dieselbe Breite aufweist, wobei die längs verlaufenden Seiten eine gemeinsame Grenze aufweisen, wobei das Übertragungselement, das Absorbens und die Zellstoffschicht eine zentrale saugfähige Zone bilden, welche mindestens etwa 90% des auf der Damenbinde abgelagerten Körperfluids absorbieren kann.

37. Damenbinde gemäß einem der Ansprüche 3 bis 36, bei der die zentrale saugfähige Zone eine Breite von weniger als 5,08 cm (2 Inch) aufweist.

## Revendications

1. Serviette hygiénique (10,56,82) ayant un axe longitudinal et comprenant :
a. une enveloppe perméable aux liquides (38,58,84) ;
b. un déflecteur imperméable aux liquides (10,60;86), et
c. un absorbant (14,62,88) étant inclus entre ladite enveloppe (38,58,84) et ledit déflecteur (12,60,86), ledit absorbant ayant une zone absorbante centrale s'étendant longitudinalement (16;72,98) alignée le long de l'axe longitudinal (x-x) de ladite serviette (10,56,82),
caractérisée en ce que
ladite zone absorbante centrale (16;72,98) a une largeur inférieure à 5,08 cm (2 pouces) et inférieure à la largeur dudit absorbant (14,62,88) mesurée au voisinage dudit axe transversal central de ladite serviette (10,56,82) et est capable d'absorber au moins 20 g de fluide corporel déposé sur ladite serviette (10:56,82),
ladite zone absorbante centrale a une valeur de rigidité qui est d'au moins 150 mg supérieure à la valeur de rigidité de portions (100,102) adjacentes à ladite zone absorbante centrale, lorsque l'on soustrait une valeur de rigidité Gurley minimale dans le sens machine de ladite zone absorbante centrale d'une valeur de rigidité Gurley maximale dans le sens machine desdites portions adjacentes (100,102) et/ou le rapport de rigidité entre ladite zone absorbante centrale et lesdites portions adjacentes (100,102) est d'environ 2:1 lorsqu'il est mesuré en divisant une valeur de rigidité Gurley moyenne dans le sens machine de ladite zone absorbante centrale par une valeur de rigidité Gurley moyenne dans le sens machine desdites portions adjacentes (100,102), et ladite serviette (10,56,82) a une épaisseur inférieure à environ 5 mm.

2. Serviette hygiénique selon la revendication 1, dans laquelle ladite zone absorbante centrale (16;72,98) a une largeur qui est inférieure à 75 % de la largeur dudit absorbant (14,62,88) mesurée au voisinage dudit axe transversal central de ladite serviette (10,56,82) et inférieure à environ 60 % de la largeur de ladite serviette lorsqu'elle est mesuré d'un côté à l'autre de la partie la plus étroite de ladite serviette.

3. Serviette hygiénique (10,56,82) ayant un axe longitudinal et comprenant :
a. une enveloppe perméable aux liquides (38, 58, 84) ;
b. un déflecteur imperméable aux liquides (10, 60 ; 86), et
c. un absorbant (14, 62, 88) étant inclus entre ladite enveloppe (38, 58, 84) et ledit déflecteur (12, 60, 86), ledit absorbant ayant une zone absorbante centrale s'étendant longitudinalement (16 ; 72,98) alignée le long de l'axe longitudinal (x-x) de ladite serviette (10,56,82),
caractérisée en ce que
ladite zone absorbante centrale (16;72,98) a une largeur inférieure à 75 % de la largeur dudit absorbant (14,62,88) mesurée au voisinage dudit axe transversal central de ladite serviette (10, 56, 82) et inférieure à 60 % de la largeur de ladite serviette lorsqu'elle est mesurée d'un côté à l'autre de la partie la plus étroite de ladite serviette, et ladite zone absorbante centrale est capable d'absorber au moins 20 g de fluide corporel déposé sur ladite serviette (10,56,82),
ladite zone absorbante centrale a une valeur de rigidité qui est d'au moins 150 mg supérieure à la valeur de rigidité de portions (100,102) adjacentes à ladite zone absorbante centrale, lorsque l'on soustrait une valeur de rigidité Gurley minimale dans le sens machine de ladite zone absorbante centrale d'une valeur de rigidité Gurley maximale dans le sens machine desdites portions adjacentes (100,102) et/ou le rapport de rigidité entre ladite zone absorbante centrale et lesdites portions adjacentes (100,102) est d'environ 2:1 lorsqu'il est mesuré en divisant une valeur de rigidité Gurley moyenne dans le sens machine de ladite zone absorbante centrale par une valeur de rigidité Gurley moyenne dans le sens machine desdites portions adjacentes (100,102), et ladite serviette (10,56,82) a une épaisseur inférieure à environ 5 mm.

4. Serviette hygiénique selon l'une des revendications 1 à 3, dans laquelle ladite zone absorbante centrale (16,72,98) a une rigidité Gurley d'au moins 500 mg.

5. Serviette hygiénique selon l'une des revendications 1 à 4, dans laquelle ladite zone absorbante centrale (16) est un stratifié (50) formé d'un superabsorbant (52) enfermé dans un non-tissé, de préférence une nappe étalée par jet d'air (54).

6. Serviette hygiénique selon l'une des revendications 1 à 5, dans laquelle ladite zone absorbante centrale est formée d'un superabsorbant (52) enfermé dans une nappe étalée par jet d'air (54).

7. Serviette hygiénique selon l'une des revendications 1 à 5, dans laquelle ladite zone absorbante centrale (16) est un stratifié (30) formé d'un superabsorbant (36) disposé entre deux nappes étalées par jet d'air (32,34).

8. Serviette hygiénique selon l'une des revendications précédentes, dans laquelle ladite zone absorbante centrale (16) est un composite (14) formé de fibres hydrophiles (22) et un superabsorbant (24).

9. Serviette hygiénique selon l'une des revendications précédentes, dans laquelle ladite zone absorbante centrale comprend une couche de superabsorbant collée à une couche de pâte de cellulose.

10. Serviette hygiénique selon l'une des revendications 5 à 9, dans laquelle ledit superabsorbant est traité à l'acide citrique pour augmenter son affinité d'absorption vis-à-vis des menstrues.

11. Serviette hygiénique selon l'une des revendications 5 à 10, dans laquelle ledit superabsorbant est un copolymêre de poly(acide acrylique) et de poly(alcool vinylique).

12. Serviette hygiénique selon l'une des revendications 5 à 10, dans laquelle ledit superabsorbant est un polymère hydroxy-fonctionnel.

13. Serviette hygiénique selon l'une des revendications 5 à 12, dans laquelle ledit superabsorbant a une valeur de capacité d'absorption sous charge d'au moins environ 20.

14. Serviette hygiénique selon l'une des revendications précédentes, dans laquelle ladite zone absorbante centrale (16,72,98) a une largeur comprise entre 1,27 cm et 5,08 cm (0,5 et 2 pouces), ladite zone s'étendant approximativement sur toute la longueur de ladite serviette et ladite serviette ayant une épaisseur inférieure à environ 4 mm.

15. Serviette hygiénique selon l'une des revendications précédentes, dans laquelle ladite zone absorbante centrale (16) est un composite formé de fibres (22) obtenues par fusion-soufflage et un superabsorbant (24).

16. Serviette hygiénique selon la revendication 15, dans laquelle ladite zone absorbante centrale (16) est un stratifié (50) formé d'un matériau hydrocolloïdal (52) enfermé dans un matériau hydrophile (54).

17. Serviette hygiénique selon la revendication 16, dans laquelle ledit matériau hydrophile est un papier absorbant mince étalé par jet d'air.

18. Serviette hygiénique selon la revendication 16 ou 17, dans laquelle ledit matériau hydrocolloïdal a une valeur de capacité d'absorption sous charge d'au moins environ 20.

19. Serviette hygiénique selon l'une des revendications précédentes, dans laquelle ledit absorbant (88) est en contact avec une couche de papier absorbant mince (96) qui est en forme de sablier.

20. Serviette hygiénique selon l'une des revendications précédentes, dans laquelle la périphérie extérieure de ladite serviette est en forme de piste de course.

21. Serviette hygiénique selon l'une des revendications précédentes, dans laquelle ladite zone absorbante centrale (98) est flanquée d'une paire de zones adjacentes (100,102) et ladite zone centrale est capable d'absorber au moins 80 %, de préférence environ 90 %, du fluide corporel déposé sur ladite serviette.

22. Serviette hygiénique selon l'une des revendications précédentes, dans laquelle ledit absorbant (16) comprend une couche de superabsorbant (44) et une couche de pâte (46) prises en sandwich entre ladite enveloppe (38) et ledit déflecteur (12).

23. Serviette hygiénique selon l'une des revendications précédentes, dans laquelle ledit absorbant comprend une couche de superabsorbant (44) lié audit déflecteur (12) et une couche fibreuse (46) fixée à la fois audit superabsorbant (44) et à ladite enveloppe (38).

24. Serviette hygiénique selon l'une des revendications précédentes, dans laquelle ladite zone absorbante centrale (98) a une épaisseur supérieure à celle desdites zones adjacentes.

25. Serviette hygiénique selon l'une des revendications précédentes, dans laquelle ladite zone absorbante centrale (98) peut absorber environ 90 % du fluide corporel déposé sur ladite serviette.

26. Serviette hygiénique selon l'une des revendications précédentes, dans laquelle ladite zone absorbante centrale (98) contient un matériau hydrocolloïdal et en ce que lesdites zones adjacentes sont dépourvues de tout matériau hydrocolloïdal.

27. Serviette hygiénique selon l'une des revendications précédentes, dans laquelle ledit absorbant est un stratifié formé d'un matériau superabsorbant pris en sandwich entre deux couches de papier absorbant mince étalées par jet d'air.

28. Serviette hygiénique selon l'une des revendications précédentes, dans laquelle ledit superabsorbant est un copolymère de poly(acide acrylique) et de poly(alcool vinylique).

29. Serviette hygiénique selon l'une des revendications précédentes, dans laquelle un élément de transfert (74,106) est disposé entre ladite enveloppe (58,84) et ledit absorbant (62,88) et est aligné le long de l'axe longitudinal (x-x) de ladite serviette, ledit élément de transfert (74,106) facilitant le mouvement du fluide corporel vers le bas et vers l'extérieur en direction de zones distantes dudit absorbant.

30. Serviette hygiénique selon l'une des revendications précédentes, dans laquelle une couche de pâte (108) est disposée entre ledit absorbant (14,63,88) et ledit déflecteur (12,60,86) et est, de préférence, alignée le long de l'axe longitudinal (x-x) de ladite serviette, ladite couche de pâte (108) offrant de la rigidité supplémentaire à ladite zone absorbante centrale pour résister à la déformation lorsque ladite serviette est pressée dans la direction transversale.

31. Serviette hygiénique selon la revendication 30, dans laquelle la couche de pâte (108) est incisée dans la direction longitudinale.

32. Serviette hygiénique selon la revendication 30 ou 31, dans laquelle ladite couche de pâte (108) a une première et une seconde surfaces principales (110,112) et dans laquelle les deux surfaces sont incisées.

33. Serviette hygiénique selon l'une des revendications précédentes, dans laquelle ledit absorbant (88) a une largeur qui est inférieure à la largeur de ladite serviette et il contient un superabsorbant.

34. Serviette hygiénique selon l'une des revendications précédentes, comprenant en outre :
d. au moins une couche de papier absorbant mince (96) enfermant ledit absorbant et s'étendant latéralement vers l'extérieur depuis ledit absorbant.
e. un élément de transfert (106) disposé entre ladite enveloppe (84) et ledit absorbant (88) et aligné le long de l'axe central longitudinal de la serviette, ledit élément de transfert (106) facilitant le mouvement du fluide corporel vers le bas et vers l'extérieur depuis ladite enveloppe vers des zones distantes dudit absorbant, et
f. une couche de pâte (108) disposée entre ledit absorbant (88) et ledit déflecteur (86) et alignée le long de l'axe longitudinal de ladite serviette, ladite couche de pâte (108) offrant de la rigidité supplémentaire audit absorbant pour résister à la déformation lorsque ladite serviette est pressée dans la direction transversale.

35. Serviette hygiénique selon l'une des revendications précédentes dans laquelle :
ladite enveloppe perméable aux liquides (84) a une paire de côtés longitudinaux, et comprend en outre :
d. au moins une couche de papier absorbant mince (96) enfermant ledit absorbant et s'étendant latéralement vers l'extérieur depuis ledit absorbant, ladite couche de papier absorbant mince ayant une forme de sablier,
e. un élément de transfert (106) disposé entre ladite enveloppe (84) et ledit absorbant (88) et aligné le long de l'axe central longitudinal de la serviette, ledit élément de transfert (106) facilitant le mouvement du fluide corporel vers le bas et vers l'extérieur depuis ladite enveloppe vers des zones distantes dudit absorbant,
f. une couche de pâte (108) positionnée entre ledit absorbant (88) et ledit déflecteur (86) et alignée le long de l'axe longitudinal de ladite serviette, ladite couche de pâte offrant de la rigidité supplémentaire audit absorbant,
g. des moyens adhésifs (121) pour fixer ladite enveloppe audit élément de transfert, à ladite couche de papier absorbant mince et audit déflecteur pour fixer ledit déflecteur à ladite couche de pâte et pour fixer ladite couche de papier absorbant mince audit absorbant et, facultativement,
h. un adhésif pour vêtements (116,118) appliqué sur ledit déflecteur (86) pour fixer ladite serviette à la portion d'entrejambe d'un sous-vêtement, et
i. une bande pelable (128) fixée de manière amovible audit adhésif pour vêtements (116,118) pour protéger ledit adhésif pour vêtements vis-à-vis des contaminations, avant sa fixation audit sous-vêtement.

36. Serviette hygiénique selon la revendication 35, dans laquelle ledit élément de transfert (106), ledit absorbant (88) et ladite couche de pâte (108) sont alignés verticalement l'un par rapport à l'autre et chacun a approximativement la même largeur avec des côtés s'étendant longitudinalement co-terminaux, ledit élément de transfert, ledit absorbant et ladite couche de pâte formant une zone absorbante centrale capable d'absorber au moins environ 90 % du fluide corporel déposé sur ladite serviette hygiénique.

37. Serviette hygiénique selon l'une des revendications 3 à 36, dans laquelle ladite zone absorbante centrale a une largeur inférieure à 5,08 cm (2 pouces).
